(19) Europäisches Patentamt
European Patent Office
Office européen des brevets

(11) Veröffentlichungsnummer: **0 001 553**
**B1**

(12) # EUROPÄISCHE PATENTSCHRIFT

(51) Int. Cl.³: C 07 C 13/40, C 07 C 33/05, C 07 C 47/45, C 07 C 69/74, C 07 C 121/48, C 11 B 9/00, // C 07 D 317/12

(21) Anmeldenummer: **78100895.8**

(22) Anmeldetag: **15.09.78**

(54) **Bicyclische Riechstoffe, Verfahren zu ihrer Herstellung und Vorprodukt**

(30) Priorität: **20.09.77 DE 2742185**

(43) Veröffentlichungstag der Anmeldung: **02.05.79 Patentblatt 79/09**

(45) Bekanntmachung des Hinweises auf die Patenterteilung: **29.10.80 Patentblatt 80/22**

(84) Benannte Vertragsstaaten: **CH DE FR GB NL**

(56) Entgegenhaltungen:
**Chemical Abstracts, Band 83, Nr. 25, 1975 Columbus, Ohio, USA, Seite 412, Spalte 1, Abstract Nr. 206 439x**

(73) Patentinhaber: **BASF Aktiengesellschaft**
**Carl - Bosch - Strasse 38**
**D - 6700 Ludwigshafen (DE)**

(72) Erfinder: **Baumann, Manfred, Dr.**
**Im Sennteich 26**
**D - 6800 Mannheim 24 (DE)**
**Hoffmann, Werner, Dr.**
**Ringstrasse 11c**
**D - 6701 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 553 B1

Bicyclische Riechstoffe, Verfahren zu ihrer Herstellung und Vorprodukt

Die Erfindung betrifft neue Verbindungen der allgemeinen Formel I

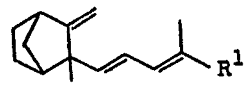

(I),

in der

$R^1$ für $—CH_2OH$; $—CHO$; $—COOCH_3$; $—COOC_2H_5$; $—CN$ oder $—CH_3$ steht,

sowie Verfahren zu deren Herstellung.

Die neuen Verbindungen besitzen interessante Riechstoffeigenschaften und stellen somit eine Bereicherung der Palette wertvoller vollsynthetischer Riechstoffe und somit auch eine Bereicherung des Standes der Technik dar.

Eine besonders interessante Duftnote besitzt die Verbindung der Formel I, in der $R^1$ für $—CH_2OH$ steht. Die neuen Verbindungen besitzen in ihrem Kohlenstoffgerüst Ähnlichkeit mit dem Gerüst eines begehrten natürlichen Duftstoffes der Sandelhölzer, dem ß-Santalol

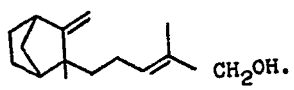

Die Verbindung der Formel I, in der R für $CH_2OH$ steht, könnte man als Dehydro-ß-santalol bezeichnen.

Die neuen Verbindungen der allgemeinen Formel I, in der $R^1$ für $—CH_3$, $—COOCH_3$ oder $COOC_2H_5$ steht, können vorteilhaft so hergestellt werden, daß man den Aldehyd der Formel II

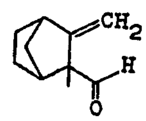

(II)

unter den Bedingungen einer Wittig-Reaktion mit einem Phosphorylid der allgemeinen Formel III

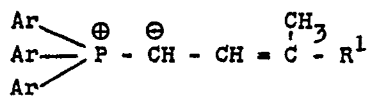

(III),

in der Ar für gleiche oder verschiedene Arylreste oder Cyclohexyl, insbesondere für Phenyl oder Tolylsteht, und $R^1$ die oben angegebene Bedeutung hat, umsetzt.

Die neue Verbindung der allgemeinen Formel I, in der $R^1$ für $—CHO$ steht, kann man dadurch erhalten, daß man den Aldehyd der Formel II

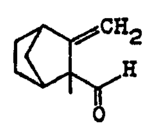

(II)

unter den Bedingungen einer Wittig-Reaktion mit einem Phosphorylid der allgemeinen Formel III

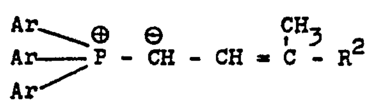

(III),

in der $R^2$ für die Acetalgruppierung

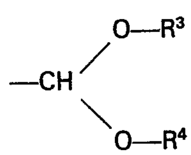

steht, in der $R^3$ und $R^4$ für einen aliphatischen Kohlenwasserstoff mit 1 bis 4 C-Atomen steht oder aber $R^3$ und $R^4$ zusammen einen Äthylenrest oder Propylenrest bedeuten, der mit einer oder mehreren Alkylgruppen mit 1 bis 4 C-Atomen, vorzugsweise Methylgruppen, substituiert sein kann und Ar für gleiche

oder verschiedene Arylreste oder Cyclohexyl, insbesondere für Phenyl oder Tolyl steht, umsetzt und das erhaltene Acetal in an sich bekannter Weise sauer hydrolysiert.

Die Verbindungen der Formel I, in der $R^1$ für $—CH_2OH$ steht, kann dadurch erhalten werden, daß man

A) den Aldehyd der Formel II

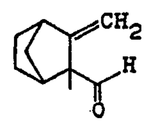

(II)

unter den Bedingungen einer Wittig-Reaktion mit einem Phosphorylid der allgemeinen Formel III

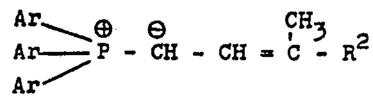

(III),

in der $R^2$ für eine Acetalgruppierung

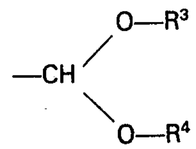

steht, in der $R^3$ und $R^4$ für einen aliphatischen Kohlenwasserstoff mit 1 bis 4 C-Atomen steht oder aber $R^3$ und $R^4$ zusammen einen Äthylenrest oder Propylenrest bedeuten, der mit einer oder mehreren Alkylgruppen mit 1 bis 4 C-Atomen, vorzugsweise Methylgruppen, substituiert sein kann und Ar für gleiche oder verschiedene Arylreste oder Cyclohexyl, insbesondere für Phenyl oder Tolyl steht, umsetzt,

B) das erhaltene Acetal in an sich bekannter Weise sauer hydrolysiert und

C) den erhaltenen Aldehyd der allgemeinen Formel I mit $LiAlH_4$ oder nach Meerwein-Ponndorf mit Aluminiumisopropylat reduziert.

Die Verbindung der Formel I, in der $R^1$ für —CN steht, kann dadurch erhalten werden, daß man den Aldehyd II

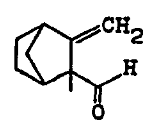

(II)

A) unter den Bedingungen einer Wittig-Reaktion mit einem Phosphorylid der allgemeinen Formel III

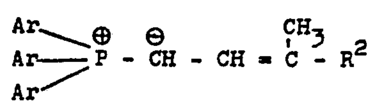

(III),

in der $R^2$ für eine Acetalgruppierung

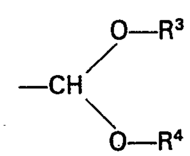

steht, in der $R^3$ und $R^4$ für einen aliphatischen Kohlenwasserstoff mit 1 bis 4 C-Atomen steht oder aber $R^3$ und $R^4$ zusammen einen Äthylenrest oder Propylenrest bedeuten, der mit einer oder mehreren Alkylgruppen mit 1 bis 4 C-Atomen, vorzugsweise Methylgruppen, substituiert sein kann und Ar für gleiche oder verschiedene Arylreste oder Cyclohexyl, insbesondere für Phenyl oder Tolyl steht, umsetzt,

B) das erhaltene Acetal in an sich bekannter Weise sauer hydrolysiert,

C) den erhaltenen Aldehyd der allgemeinen Formel I in an sich bekannter Weise mit einem Hydroxylammoniumsalz in das Oxim überführt und

D) das erhaltene Oxim in an sich bekannter Weise dehydratisiert.

Der wesentliche gemeinsame Reaktionsschritt der Herstellung der neuen bicyclischen Riechstoffe liegt in der Wittig-Reaktion zwischen dem Aldehyd der Formel II und einem Phosphorylid der allgemeinen Formel III.

Der Aldehyd II (2-Methylen-3-methyl-3-formyl-bicyclo-[2,2,1]-heptan ist bisher weder synthetisiert noch charakterisiert worden. In Helv. Chim. Acta *59* (1976), S. 738 heißt es, daß einige experimentelle Anzeichen vermuten lassen, daß das bicyclische Teresantalal II auch im Sandelholzöl vorkommt. Der Aldehyd II kann aus dem neuen, jedoch in der nicht vorveröffentlichten Patentanmeldung P 27 19 976 beschriebenen 2-Chlormethyl-3-methyl-3-formyl-bicyclo-[2,2,1]-heptan-(5) durch HCl-Abspaltung unter Schutz der Formylgruppe durch Acetalisieren erhalten werden. Er hat einen Siedepunkt von 74 bis 78°C bei einem Druck von 26 Pa (0,2 Torr).

Die Phosphorylide können in an sich bekannter Weise, z.B. durch Einwirken starker Basen auf die ihnen zugrundeliegenden Phosphoniumsalze hergestellt werden. Näheres hierüber ist u.a. der zusammenfassenden Arbeit von Tripett (Quart. Reviews, Band *17* (1963), Seiten 406 ff) zu entnehmen.

Für die erfindungsgemäße Umsetzung ist es zweckmäßig, daß man das Phosphorylid gleich in dem für die Wittg-Synthese vorgesehenen Lösungsmittel und gegebenenfalls sogar in Gegenwart des umzusetzenden Aldehyds aus dem entsprechenden Phosphoniumsalz herstellt. Die benötigten Phosphoniumsalze können in an sich bekannter und einfacher weise durch Umsetzung der entsprechenden Alkylhalogenide, insbesondere den entsprechenden Alkylchloriden mit den Triaryl (Tricyclohexyl)-phosphinen, insbesondere mit Triphenylphosphin hergestellt werden.

Als starke Basen zur Herstellung der Phosphoryliie können die für Wittig-Synthesen üblichen Basen verwende werden. Genannt seien Alkalihydroxide, Alkalihydride, Alkaliamide und Alkali- und Erdalkalialkoholate, Phenyl-Lithium und Butyl-Lithium, vorzugsweise Natrium- und Kaliumalkoholate.

Aus Äthylenoxid (siehe Angew. Chem. *80* (1968), Seite 535 ff.) und überschüssiges Phosphorylid können unter bestimmten Bedingungen die Rolle der starken Base übernehmen.

Als Lösungsmittel für die Herstellung der Phosphorylide sowie für die Durchführung der Wittig-Reaktion kommen die für Wittig-Synthesen üblichen Lösungsmittel, beispielsweise aliphatische oder aromatische Kohlenwasserstoffe, wie Hexan, Octan, Cyclohexan, Benzol, Toluol und Xylol und deren Halogenierungsprodukte, ferner Alkohole, wie Methanol, Äthanol, Isopropanol, Butanole, Hexanole, Cyclohexanol und Cyclooctanol, auch Glykole, sowie Äther, wie Diisopropyläther, Äthylenglykoldimethyläther, Tetrahydrofuran, Dimethyltetrahydrofuran und Dioxan oder Gemische aus ihnen in Frage. Besonders geeignet sind polare organische Lösungsmittel, wie Methanol, Äthanol, Formamid, Dimethylforamid, N-Methylpyrrolidon, Hexamethylphosphorsäuretriamid, Acetonitril und Dimethylsulfoxid oder Gemische dieser Lösungsmittel. Auch in Wasser oder in Wasser enthaltenden Gemischen läßt sich das Verfahren der Erfindung ausführen.

Zur Durchführung der erfindungsgemäßen Umsetzung des Aldehyds II mit den Phosphoryliden III werden zweckmäßig Phosphoniumsalze der Formel IV

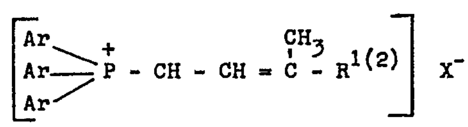

(IV),

in der

X für Chlor, Brom oder Jod steht und $R^1$ bzw. $R^2$ und Ar die oben angegebene Bedeutung haben, und etwa stöchiometrische Mengen des umzusetzenden Aldehyds in einem Lösungsmittel vorgelegt und zu dieser Suspension unter Rühren, gegebenenfalls Kühlen und Einleiten von trockenem Stickstoff bei Temperaturen von −20 bis +70°C, vorzugsweise 0 bis 30°C, portionsweise etwa stöchiometrische Mengen einer starken Base eingetragen. Anschließend wird das Reaktionsgemisch noch 1 bis 24 Stunden, vorzugsweise 1 bis 2 Stunden, auf Temperaturen von 15 bis 30°C, vorzugsweise Raumtemperatur, gehalten.

Man kann die erfindungsgemäße Umsetzung aber auch so durchführen, daß man zu der Lösung der Phosphoniumsalze bei den oben genannten Temperaturen etwa stöchiometrische Mengen einer starken Base gibt, zu der so erhaltenen Lösung der Phosphorylide den Aldehyd der Formel II addiert und dann das Reaktionsgemisch wie oben beschrieben ausreagieren läßt.

Insgesamt werden für diese Umsetzung etwa 1 bis 1,2 Mol Base pro Mol Phosponiumsalze verwendet. Die Aufarbeitung des Reaktionsgemisches erfolgt auf übliche Weise durch Abtrennen der Reaktionsprodukte von dem entstandenen Triaryl(Tricyclohexyl)phosphinoxid, beispielsweise durch Extraktion, Destillation und gegebenenfalls anschließende präparative Chromatographie.

Die saure Hydrolyse der bei der Wittig-Reaktion des Aldehyds II mit den eine Acetalgruppierung enthaltenden Phosphoryliden III erfolgt auf an sich bekannte Weise. Beispielsweise werden die Acetale zweckmäßig mit 0,01 bis 1 Mol einer Mineralsäure, wie Schwefelsäure oder Salzsäure, oder einer organischen Säure, wie Ameisensäure, p-Toluolsulfonsäure oder Essigsäure, pro Mol Acetal in Form einer 1- bis 20-prozentigen Lösung versetzt und unter intensiver Vermischung 0,5 bis 5, vorzugsweise 2 bis 3 Stunden auf Temperaturen von 10 bis 50°C erhitzt.

Bei der Hydrolyse empfiehlt sich der Zusatz eines Lösungsvermittlers zum Reaktionsgemisch. Als Lösungsvermittler sind niedere aliphatische Alkohole, wie Methanol, Äthanol und Propanol sowie

cycloaliphatische Äther, wie Tetrahydrofuran und Dioxan besonders geeignet. Die erhaltenen Aldehyde können auf übliche Weise, beispielsweise durch Extraktion nach schonender Neutralisation des Reaktionsgemisches, z.B. mit einem Alkalibicarbonat oder Natriumcarbonat, und Abdestillieren des Extraktionsmittels isoliert werden.

Der Aldehyd der Formel I kann in an sich bekannter Weise mit $LiAlH_4$, $NaBH_4$ oder Aluminiumisopropylat zu dem Alkohol der Formel I reduziert werden.

Zur Umsetzung mit $LiAlH_4$ verwendet man als Lösungsmittel vorzugsweise ätherische Lösungsmittel, wie Diäthyläther oder Tetrahydrofuran (THF). Die Lösungsmittel verwendet man zweckmäßig in Mengen von etwa 1/4 bis 1 Mol pro Mol Aldehyd. Die Reaktionstemperatur beträgt im allgemeinen Raumtemperatur bis Rückflußtemperatur des Lösungsmittels. Die Reaktionszeit beträgt etwa 1 bis 6 Stunden.

$LiAlH_4$ wird in einem wasserfreien Lösungsmittel (teilweise gelöst, teilweise suspendiert) vorgelegt.

Zu dieser Mischung wird die Carbonylverbindung in den betreffenden Lösungsmitteln langsam zugefügt. Je nach Reaktivität der Carbonylverbindung ist die Reaktion nach mehrstündigem Rühren bei Raumtemperatur beendet oder aber das Reaktionsgemisch wird einige Stunden erhitzt.

Überschüssiges $LiAlH_4$ wird durch vorsichtiges Zugeben eines Alkohols, beispielsweise Äthanol, zerstört. Die organische Phase wird mit Wasser ausgewaschen, getrocknet und eingeengt. Das Reaktionsprodukt wird auf übliche Weise, beispielsweise durch Destillation, gereinigt.

Zur Umsetzung mit $NaBH_4$ verwendet man im allgemeinen protische Lösungsmittel, wie beispielsweise Wasser, Äthanol, vorzugsweise Äthanol. Zweckmäßig verwendet man das Lösungsmittel in einer Menge von etwa 1/4 bis 1 Mol pro Carbonylverbindung. Die Reaktionstemperatur ist im allgemeinen Raumtemperatur; die Reaktionsdauer beträgt etwa 2 bis 12 Stunden.

Die Aufarbeitung des Reaktionsgemisches erfolgt auf übliche Weise, beispielsweise durch Zersetzen des Rückstandes mit verdünnten Säuren und anschließender Extraktion mit einem Lösungsmittel.

Zur Reduktion des Aldehyds der Formel I mit Aluminiumisopropylat (Meerwein - Ponndorf - Verley - Reduktion) verwendet man als Lösungsmittel wasserfreien Isopropylalkohol. Zur Durchführung der Reduktion wird das Al-isopropylat in Isopropanol vorgelegt und die Carbonylverbindung zu dieser Mischung zugegeben. Man verwendet das Al-Isopropylat in Mengen von 1/3 bis 2 Mol pro Mol Carbonylverbindung. Durch Erhitzen der Mischung entsteht Aceton, das über eine Kolonne abdestilliert wird. Die Reaktionstemperatur beträgt im allgemeinen etwa 60 bis 120°C; die Reaktionszeit etwa 30 bis 60 Minuten.

Wenn kein Aceton mehr entsteht, wird der Rückstand mit verdünnter Säure zersetzt und das Produkt beispielsweise durch Extraktion mit einem Lösungsmittel isoliert.

Zur Überführung des Aldehyds der Formel I in das Oxim versetzt man zweckmäßig die Carbonylverbindung mit der äquimolaren Menge oder einem geringen Überschuß eines Hydroxylammoniumsalzes, wie Hydroxylammoniumchlorid oder Hydroxylammoniumsulfat in Wasser oder Alkohol oder einem Gemisch aus Alkohol und Wasser.

Die Reaktionstemperatur liegt im allgemeinen zwischen Raumtemperatur und Siedetemperatur des Lösungsmittels. Um die freiwerdende Säure abzufangen, werden Basen, wie Hydroxide, Carbonate, Hydrogencarbamate, Acetate oder dergleichen zugefügt, um einen pH-Wert des Reaktionsgemisches von etwa 4 bis 5 zu erreichen.

Zur Aufarbeitung wird mit Wasser versetzt und das Oxim durch Extraktion mit einem Lösungsmittel isoliert.

Auch die Dehydratisierung des Oxims erfolgt in an sich bekannter Weise durch Umsetzen mit wasserentziehenden Mitteln, wie Anhydriden, Säurechloriden, $P_2O_5$ oder durch thermische Wasserabspaltung. Zweckmäßig erhitzt man das Oxim mehrere Stunden mit einem großen Überschuß von Acetanhydrid. Die Reaktionstemperatur liegt im allgemeinen zwischen Raumtemperatur und dem Siedepunkt des jeweiligen Lösungsmittels. Die Reaktionsdauer beträgt etwa 1 bis mehrere Stunden. Als Lösungsmittel werden entweder das Oxim selbst oder aber aprotische Lösungsmittel, wie chlorierte Kohlenwasserstoffe, $CH_2Cl_2$ oder $CHCl_3$; Kohlenwasserstoffe, wie Toluol oder Xylol; oder aber Äther, wie Dioxan oder Tetrahydrofuran verwendet. Die Aufarbeitung erfolgt im allgemeinen durch Zersetzung mit Wasser und Extraktion mit einem Lösungsmittel.

Die neuen bicyclichen Verbindungen zeichnen sich durch interessante Riechstoffeigenschaften aus und sie erweitern dadurch die Palette wertvoller vollsynthetischer Duftstoffe.

## BEISPIEL 1.

73g (0,2 Mol) 3-Methyl-2-buten-1-triphenylphosphoniumchlorid werden in 200ml THF suspendiert. Unter Kühlung wird die 1,1-molare Menge einer 1,6n Butyllithium-Lösung (138ml) in Hexan zugetropft. Der Kolbeninhalt verfärbt sich tief rot. Anschließend werden 29g (0,2 Mol) des Aldehyd 2-Methylen-3-methyl-3-formyl-bicyclo-[2,2,1]-heptan in 50ml THF zugetropft und das Reaktionsgemisch 6 Stunden auf 50°C erhitzt.

Anschließend wird die Lösung eingeengt, der Rückstand mit petroläther ausgekocht und die

Petrolätherlösung mit wäßrigem Methanol gewaschen. Es wird getrocknet und eingeengt. Es hinterbleibt ein Rückstand von 38,6g.

Bei der anschließenden Destillation erhält man neben 3g Ausgangsaldehyd 30,6g 2-Methylen-3-methyl-3-(4'-methyl-1',3'-pentadien-1'-yl)-bicyclo-[2,2,1]-heptan (II) vom Siedepunkt Kp = 65°C bei einem Druck von 1,33 Pa (0,01 Torr); $n_D^{25}$ = 1,5260; IR- und NMR-Spektren bestätigen die Struktur; das NMR-Spektrum zeigt ein Gemisch aus exo- und endo-Produkt. Ausbeute 82% bezogen auf umgesetzten Aldehyd. Geruchsbeurteilung: angenehm holzig.

BEISPIEL 2

85g (0,2 Mol) 3-Äthoxycarbonyl-2-buten-1-triphenylphosphoniumchlorid werden analog Beispiel 1 mit 138ml einer 1,6n Butyllithium-Lösung (entsprechend der 1,1-molaren Menge) in Hexan und dann mit 29g (0,2 Mol) 2-Methylen-3-methyl-3-formyl-bicyclo-[2,2,1]-heptan umgesetzt. Man erhält neben 14,3g unumgesetztem Aldehyd 6,2g 2-Methylen-3-methyl-3-(4'-äthoxycarbonyl-1',3'-pentadien-1'-yl)-bicyclo-[2,2,1]-heptan vom Siedepunkt Kp = 150°C bei 1,33 Pa (0,01 Torr); $n_D^{25}$ = 1,5312. Ausbeute 24%, bezogen auf umgesetzten Aldehyd.

Geruchsbeurteilung: süß, fruchtig, anisig.

BEISPIEL 3

a) 90,5g (0,2 Mol) 3-(4'-Methyl-1',3'-dioxan-2'-yl)-2-buten-1-triphenylphosphoniumchlorid werden analog Beispiel 1 mit 138ml einer 1,6n Butyllithium-Lösung in Hexan und dann mit 29g des Aldehyds II umgesetzt. Man erhält neben 19g unumgesetztem Aldehyd 18,6g 2-Methylen-3-methyl-3-[4'-(4"-methyl-1",3"-dioxan-2"-yl)1',3'-pentadien-1'-yl]-bicyclo-[2,2,1]-heptan vom Siedepunkt Kp = 160°C bei 1,33 Pa (0,01 Torr); $n_D^{25}$ = 1,5233. IR- und NMR-Spektren bestätigen die Struktur. Ausbeute 93%, bezogen auf umgesetzen Aldehyd.

b) 27g des gemäß 3 a) erhaltenen Acetals werden zusammen mit 50ml einer 10-prozentigen wäßrigen $H_2SO_4$ und 50ml Dioxan 2 Stunden bei 20°C gerührt. Anschließend wird das Reaktionsgemisch mit 50ml Wasser verdünnt und mit Äther extrahiert. Die Ätherextrakte werden mit Bicarbonatlösung neutral gewaschen, getrocknet und eingeengt.

Man erhält 10g 2-Methylen-3-methyl-3-(4'-formyl-1',3'-pentadien-1'-yl)-bicyclo-[2,2,1]-heptan vom Siedepunkt Kp = 103 bis 106°C bei 26,6 Pa (0,2 Torr); IR- und NMR-Spektren bestätigen die Struktur. Ausbeute 50%, bezogen auf eingesetzten Aldehyd II.

Geruchsbeurteilung: frisch, holzig.

BEISPIEL 4

9,2g (0,0426 Mol) des gemäß 3b) erhaltenen Aldehyds werden in 20ml Äthanol gelöst und zu einer Mischung aus 0,6g (0,015 Mol) $NaBH_4$ und 50ml Äthanol getropft. Das Reaktionsgemsich wird 4 Stunden bei 20°C nachgerührt, anschließend eingeengt, mit Wasser versetzt, mit verdünnter $H_2SO_4$ schwach angesäuert und mit Äther extrahiert. Die erhaltene organische Phase wird getrocknet und eingeengt. Bei der anschließenden Destillation erhält man 6,4g 2-Methylen-3-methyl-3-(4'-hydroxymethyl-1',3'-pentadien-1'-yl)-bicyclo-[2,2,1]-heptan vom Siedepunkt Kp = 130°C bei 10,67 Pa (0,08 Torr). Das Produkt erstarrt im Kühlschrank wachsartig. IR- und NMR-Spektren bestätigen die Struktur. Ausbeute 70%, bezogen auf eingesetztes Acetal.

Geruchsbeurteilung: balsamisch, holzig, mild.

BEISPIEL 5

9g des gemäß 3 b) erhaltenen bicyclischen Aldehyds werden mit 6g Hydroxylammoniumchlorid, 6g Na-acetat und 40g Wasser versetzt und das erhatlene Gemisch 30 Minuten auf 60°C erwärmt. Anschließend wird das Reaktionsgemisch abgekühlt, mit 50ml Wasser versetzt, mit Äther extrahiert und die vereinigten Ätherphasen eingeengt. Das erhaltene rohe Oxim wird mit 20g Acetanhydrid versetzt und das erhaltene Gemisch 2 Stunden unter Rückfluß zum Sieden erhitzt. Anschließend wird mit Wasser versetzt, mit Äther extrahiert, die erhaltene Ätherphase neutralisiert, getrocknet und eingeent. Der Rückstand wird durch Chromatographie an Kieselgel mit Petroläther/Äther (3 : 1) als Laufmittel gereinigt. Man erhält 5,4g 2-Methylen-3-methyl-3-(4'-cyano-1',3'-pentadien-1'-yl)-bicyclo-[2,2,1]-heptan vom Siedpunkt Kp = 100 bis 105°C bei 26,66 Pa (0,2 Torr). Ausbeute 61%, bezogen auf eingesetzten Aldehyd. Der Geruch ähnelt dem des Aldehyds der Formel I, ist aber etwas fruchtiger.

**Patentansprüche:**

1. Bicyclische Riechstoffe der allgemeinen Formel I

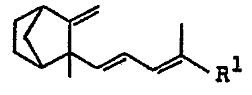

(I),

in der
$R^1$ für $-CH_2OH$; $-CHO$; $-COOCH_3$; $-COOC_2H_5$; $-CN$ oder $-CH_3$ steht.

2. Verfahren zur Herstellung von Verbindungen der Formel I gemäß Anspruch 1, in der $R^1$ für $-CH_3$; $-COOCH_3$ oder $-COOC_2H_5$ steht, *dadurch gekennzeichnet,* daß man den Aldehyd der Formel II

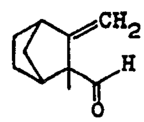
(II)

unter den Bedingungen einer Wittig-Reaktion mit einem Phosphorylid der allgemeinen Formel III

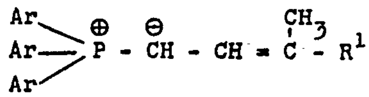
(III),

in der $R^1$ die oben angegebene Bedeutung hat und Ar für gleiche oder verschiedene Arylreste oder Cyclohexyl, insbesondere für Phenyl oder Tolyl steht, umsetzt.

3. Verfahren zur Herstellung der Verbindung der Formel I gemäß Anspruch 1, in der $R^1$ für $-CHO$ steht, *dadurch gekennzeichnet,* daß man

A) den Aldehyd der Formel II

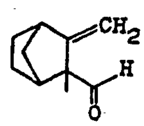
(II)

unter den Bedingungen einer Wittig-Reaktion mit einem Phosphorylid der allgemeinen Formel III

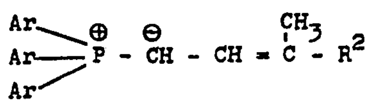
(III)

in der $R^2$ für eine Acetalgruppierung

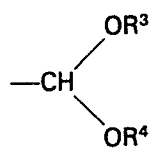

steht, in der $R^3$ und $R^4$ für einen aliphatischen Kohlenwasserstoffrest mit 1 bis 4 C-Atomen steht oder aber $R^3$ und $R^4$ zusammen einen Äthylenrest oder Propylenrest bedeuten, der mit einer oder mehreren Alkylgruppen mit 1 bis 4 C-Atomen, vorzugsweise Methylgruppen substituiert sein kann und Ar für gleiche oder verschiedene Arylreste oder Cyclohexyl, insbesondere für Phenyl oder Tolyl steht, umsetzt und

B) das erhaltene Acetal in an sich bekannter Weise sauer hydrolysiert.

4. Verfahren zur Herstellung der Verbindung der Formel I gemäß Anspruch 1, in der $R^1$ für $-CH_2OH$ steht, *dadurch gekennzeichnet,* daß man

A) den Aldehyd der Formel I

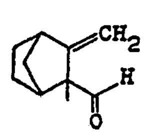
(II)

unter den Bedingungen einer Wittig-Reaktion mit einem Phosphorylid der allgemeinen Formel III

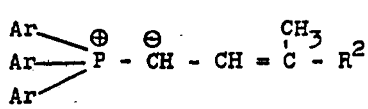
(III),

in der $R^2$ für eine Acetalgruppierung

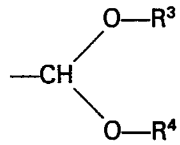

steht, in der $R^3$ und $R^4$ für einen aliphatischen Kohlenwasserstoffrest mit 1 bis 4 C-Atomen steht oder aber $R^3$ und $R^4$ zusammen einen Äthylenrest oder Propylenrest bedeuten, der mit einer oder mehreren Alkylgruppen mit 1 bis 4 C-Atomen, vorzugsweise Methylgruppen substituiert sein kann und Ar für gleiche oder verschiedene Arylrest oder Cyclohexyl, insbesondere für Phenyl oder Tolyl steht, umsetzt,

B) das erhaltene Acetal in an sich bekannter Weise sauer hydrolysiert und

C) den erhaltenen Aldehyd der allgemeinen Formel I mit $LiAlH_4$, $NaBH_4$ oder nach Meerwein-Ponndorf mit Aluminiumisopropylat reduziert.

5. Verfahren zur Herstellung der Verbindung der Formel I gemäß Anspruch 1, in der $R^1$ für —CN steht, *dadurch gekennzeichnet,* daß man

A) den Aldehyd der Formel II

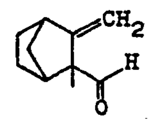

unter den Bedingungen einer Wittig-Reaktion mit einem Phosphorylid der allgemeinen Formel III

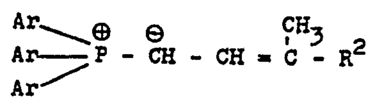

(III),

in der $R^2$ für eine Acetalgruppierung

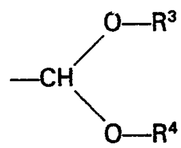

steht, in der $R^3$ und $R^4$ für einen aliphatischen Kohlenwasserstoffrest mit 1 bis 4 C-Atomen steht oder aber $R^3$ und $R^4$ zusammen einen Äthylenrest oder Propylenrest bedeuten, der mit einer oder mehreren Alkylgruppen mit 1 bis 4 C-Atomen, vorzugsweise Methylgruppen substituiert sein kann und Ar für gleiche oder verschiedene Arylreste oder Cyclohexyl, insbesondere für Phenyl oder Tolyl steht, umsetzt

B) das erhaltene Acetal in an sich bekannter Weise sauer hydrolysiert,

C) den erhaltenen Aldehyd der allgemeinen Formel I in an sich bekannter Weise mit einem Hydroxylammoniumsalz in das Oxim überführt und

D) das erhaltene Oxim in an sich bekannter Weise dehydratisiert.

6. 2-Methylen-3-methyl-3-formyl-bicyclo-[2,2,1]-heptan als neues Vorprodukt.

**Claims:**

1. Bicyclic perfume compounds of the general formula I

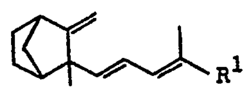

(I),

where $R^1$ denotes $—CH_2OH$, —CHO, $—COOCH_3$, $—COOC_2H_5$, —CN or $—CH_3$.

2. A process for the manufacture of compounds of the formula I according to claim 1, where $R^1$ denotes $—CH_3$, $—COOCH_3$ or $—COOC_2H_5$, *characterized in that* the aldehyde of the formula II

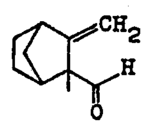

(II),

is reacted, under the conditions of a Wittig reaction, with a phosphorylide of the general formula III

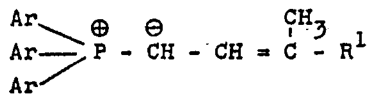

(III),

where $R^1$ has the meanings given above and Ar denotes identical or different aryl radicals or cyclohexyl, especially phenyl or tolyl.

3. A process for the manufacture of a compound of the formula I according to claim 1, where $R^1$ denotes —CHO, *characterized in that*

A) the aldehyde of the formula II

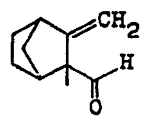

(II)

is reacted, under the conditions of a Wittig reaction, with a phosphorylide of the general formula III

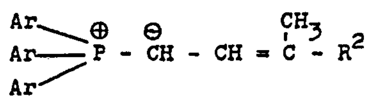

(III)

where $R^2$ denotes an acetal grouping

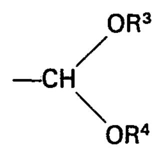

where $R^3$ and $R^4$ each denote an aliphatic hydrocarbon radical of 1 to 4 carbon atoms or $R^3$ and $R^4$ together denote an ethylene or propylene radical which may be substituted by one or more alkyl groups of 1 to 4 carbon atoms, preferably methyl, and Ar denotes identical or different aryl radicals or cyclohexyl, especially phenyl or tolyl, and

B) the resulting acetal is subjected to acid hydrolysis in a conventional manner.

4. A process for the manufacture of a compound of the formula I according to claim 1, where $R^1$ denotes —$CH_2OH$, *characterized in that*

A) the aldehyde of the formula II

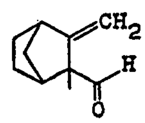

(II)

is reacted, under the conditions of a Wittig reaction, with a phosphorylide of the general formula III

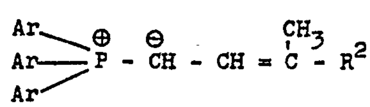

(III),

where $R^2$ denotes an acetal grouping

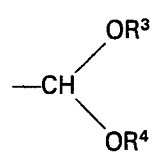

where $R^3$ and $R^4$ each denote an aliphatic hydrocarbon radical of 1 to 4 carbon atoms or $R^3$ and $R^4$ together denote an ethylene or propylene radical which may be substituted by one or more alkyl groups of 1 to 4 carbon atoms, preferably methyl, and Ar denotes identical or different aryl radicals or cyclohexyl, especially phenyl or tolyl,

B) the resulting acetal is subjected to acid hydrolysis in a conventional manner, and

C) the resulting aldehyde of the general formula I is reduced with $LiAlH_4$, $NaBH_4$ or, according to Meerwein-Ponndorf, with aluminium isopropylate.

5. A process for the manufacture of a compound of the formula I according to claim 1, where $R^1$ denotes —CN, *characterized in that*

A) the aldehyde of the formula II

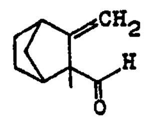

(II)

is reacted, under the conditions of a Wittig reaction, with a phosphorylide of the general formula III

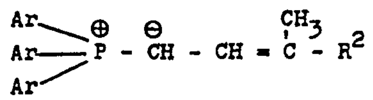

(III),

where $R^2$ denotes an acetal grouping

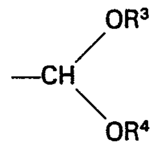

where $R^3$ and $R^4$ each denote an aliphatic hydrocarbon radical of 1 to 4 carbon atoms or $R^3$ and $R^4$ together denote an ethylene or propylene radical which may be substituted by one or more alkyl groups of 1 to 4 carbon atoms, preferably methyl, and Ar denotes identical or different aryl radicals or cyclohexyl, especially phenyl or tolyl,

B) the resulting acetal is subjected to acid hydrolysis in a conventional manner,

C) the resulting aldehyde of the general formula I is converted into the oxime with a hydroxylammonium salt in a conventional manner, and

D) the oxime obtained is dehydrated in a conventional manner.

6. 2-Methylene-3-methyl-3-formyl-bicyclo-[2,2,1]-heptane is a novel intermediate.

**Revendications:**

1. Matières odorantes bicycliques de formule générale I

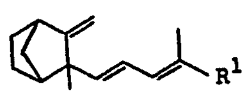

(I),

dans laquelle

$R^1$ désigne —$CH_2OH$; —CHO; —$COOCH_3$; —$COOC_2H_5$; —CN ou —$CH_3$.

2. Procédé de préparation de composés de formule I selon la revendication 1, formule dans laquelle $R^1$ désigne —$CH_3$, —$COOCH_3$ ou —$COOC_2H_5$, caractérisé par le fait qu'on fait réagir l'aldéhyde de formule II

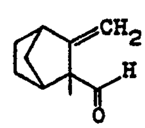

(II),

dans les conditions d'une réaction de Wittig, avec un phosphorylide de formule générale III

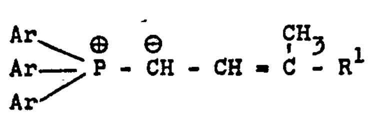

(III),

dans laquelle $R^1$ a la signification indiquée plus haut et Ar représente des restes aryle identiques ou différents ou le radical cyclohexyle, en particulier des restes phényle ou tolyle.

3. Procédé de préparation du composé de formule I, selon la revendication 1, formule dans laquelle $R^1$ désigne —CHO, caractérisé par le fait qu'on fait réagir

A) dans les conditions d'une réeaction de Wittig, l'aldéhyde de formule II

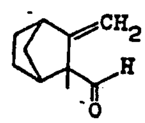

(II),

avec un phosphorylide de formule générale III

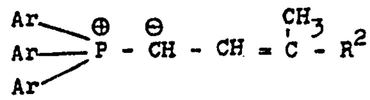

(III),

dans laquelle $R^2$ désigne le groupement acétal

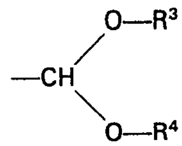

dans lequel $R^3$ et $R^4$ représentent un hydrocarbure aliphatique en $C_1$ à $C_4$ ou forment ensemble un reste éthylène ou propylène pouvant être substitué par un ou plusieurs groupes alkyle en $C_1$ à $C_4$, en particulier des groupes méthyle, Ar représentant des restes aryle identiques ou différents ou le radical cyclohexyle, en particulier des restes phényle ou tolyle, et

B) on soumet l'acétal ainsi obtenu, de façon connue en soi, à une hydrolyse acide.

4. Procédé de préparation du composé de formule I, selon la revendication 1, formule dans laquelle $R^1$ désigne $—CH_2OH$, caractérisé par le fait qu'on fait réagir

A) dans les conditions d'une réaction de Wittig, l'aldéhyde de formule II

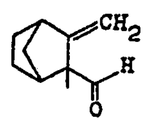

(II),

avec un phosphorylide de formula générale III

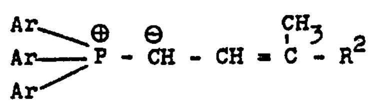

(III),

dans laquelle $R^2$ désigne le groupement acétal

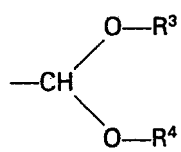

dans lequel $R^3$ et $R^4$ représentent un hydrocarbure aliphatique en $C_1$ à $C_4$ ou forment ensemble un reste éthylène ou propylène pouvant être substitué par un ou plusieurs groupes alkyle en $C_1$ à $C_4$, en particulier des groups méthyle, Ar représentant des restes aryle identiques ou différents ou le radical cyclohexyle, en particulier des restes phényle ou tolyle, et

B) on soumet l'acétal ainsi obtenu, de façon connue en soi, à une hydrolyse acide,

C) on réduit l'aldéhyde ainsi obtenu, de formule générale I, avec $LiAlH_4$, $NaBH_4$ ou, suivant Meerwein-Ponndorf, avec l'isopropylate d'aluminium.

5. Procédé de préparation du composé de formule I, selon la revendication 1, formule dans laquelle $R^1$ désigne —CN, caractérisé par le fait qu'on fait réagir

A) dans les conditions d'une réaction de Wittig, l'aldéhyde de formule II

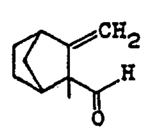

(II),

avec un phosphorylide de formule générale III

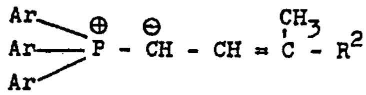 (III),

dans laquelle $R^2$ désigne le groupement acétal

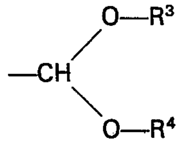

dans lequel $R^3$ et $R^4$ représentent un hydrocarbure aliphatique en $C_1$ à $C_4$ ou forment ensemble un reste éthylène ou propylène pouvant être substitué par un ou plusieurs groupes alkyle en $C_1$ à $C_4$, en particulier des groupes méthyle, Ar représentant des restes aryle identiques ou différents ou le radical cyclohexyle, en particulier des restes phényle ou tolyle, et

B) on soumet l'acétal ainsi obtenu, de façon connue en soi, à une hydrolyse acide,

C) on transforme l'aldéhyde ainsi obtenu, de formule générale I, de façon connue en soi en oxime par réaction avec un sel d'hydroxylammonium, et

D) on déshydrate l'oxime résultante de façon connue en soi.

6. Le 2-méthylène-3-méthyl-3-formyl-bicyclo-[2,2,1]-heptane en tant que nouveau produit de base.